Europäisches Patentamt

European Patent Office

Office européen des brevets

(19)

(11) **EP 1 550 463 A1**

(12) **EUROPEAN PATENT APPLICATION**
published in accordance with Art. 158(3) EPC

(43) Date of publication:
06.07.2005 Bulletin 2005/27

(21) Application number: 03797684.2

(22) Date of filing: 19.09.2003

(51) Int Cl.7: **A61K 48/00**, A61K 31/7088,
A61K 47/42, A61K 9/08,
A61K 9/14, A61P 43/00

(86) International application number:
**PCT/JP2003/011962**

(87) International publication number:
**WO 2004/026343 (01.04.2004 Gazette 2004/14)**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: 20.09.2002 JP 2002274926

(71) Applicants:
• **Sumitomo Pharmaceuticals Company, Limited
Osaka-shi, Osaka 541-8510 (JP)**
• **KOKEN CO., LTD.
Toshima-ku, Tokyo 171-0031 (JP)**

(72) Inventors:
• **ANDO, Yukio
Kumamoto-shi, Kumamoto 861-5514 (JP)**
• **NAKAMURA, Masaaki
Kumamoto-shi, Kumamoto 860-0075 (JP)**
• **NAGAHARA, Shunji
Ibaraki-shi, Osaka 567-0841 (JP)**

(74) Representative: **VOSSIUS & PARTNER
Siebertstrasse 4
81675 München (DE)**

(54) **PREPARATION FOR FACILITATING SITE-SPECIFIC GENE CONVERSION AND PREPARATION FOR GENE THERAPY**

(57)    It is intended to provide a preparation whereby an oligonucleotide is efficiently transferred into a cell and the localization thereof in nucleus is promoted, a preparation for facilitating the conversion of the base sequence of a target genomic gene, and a preparation for gene therapy. Namely, a preparation for facilitating site-specific gene conversion which comprises at least a collagen and an oligonucleotide for gene conversion; a preparation for site-specific gene therapy which comprises at least a collagen and an oligonucleotide for gene conversion; a method of arbitrarily converting a specific base on a genomic gene in the nucleus of a cell which comprises bringing the above-described preparation for facilitating gene conversion into contact with the cell; and an preparation for facilitating oligonucleotide intranuclear localization which comprises at least a collagen and an oligonucleoitde.

Fig 2

(a) DNA oligonucleotide embedded in 0.01% atelocollagen
(b) DNA oligonucleotide embedded in 0.1% atelocollagen
(c) DNA oligonucleotide encapsulated in HVJ–liposome

**Description**

TECHNICAL FIELD

The present invention relates to new utility of collagen, more particularly, a preparation for facilitating site-specific gene conversion of a genome gene comprising a collagen and an oligonucleotide and the like.

BACKGROUND ART

Gene therapy is greatly expected as a method of fundamentally treating a gene disease which is developed due to mutation or deletion of a gene. Generally, in gene therapy, a procedure of introducing a gene encoding a protein necessary for therapy into a cell using a virus vector, a liposome vector or a plasmid DNA vector, to incorporate the gene into a genome gene of a cell, or a procedure of making the gene reside with a genome gene to express a protein has been tried, but there is a few of examples in which satisfactory therapeutic effect is obtained. The cause is thought as follows: 1) it is difficult to incorporate a gene of a large size encoding a whole protein into a virus vector or a plasmid DNA to express it, 2) when an adenovirus vector or a plasmid DNA vector not incorporating an introduced gene into a genome gene is used, stable long term expression is not obtained, 3) since a retrovirus vector incorporates an introduced gene into an unspecified position of a genome gene, there is rather a possibility that function of a normal gene is lost, 4) when a virus vector is used, a virus-derived protein is produced, an immunological reaction to this protein is induced, and side-effect is produced, 5) further, since a promoter has high specificity for a cell, a cell which can express a gene is limited (Li-Wen Lai et al., "Experimental Nephrology" 1999, vol.7, p.11-14).

On the other hand, in a gene disease, it is rare that all genes of necessary proteins are completely deleted, and since only one base on a gene is erroneous in many cases, it is substituted with a different amino acid, and since one base is deleted or inserted, frame shift occurs, and a normal protein is not produced, this is a cause therefor. For example, familial amyloidotic polyneuropathy (FAP) has a precursor protein of transthyretin (TTR), apolipoprotein AI, or gelsolin which has been gene-mutated, and is one of systemic amyloidosises leading to amyloid sedimentation in various organs and tissues. Among them, FAP type I (FAP ATTR Val30Met), where atypical TTR in which 30th valine of TTR, composed of 127 amino acids is mutated into methionine becomes amyloid and organ disorder is caused, is a genetic amyloidosis exhibiting an autosome dominant inheritance having, as main symptom, multiple neuritis accompanied with limb sensory disorder and motor nerve disorder, autonomic disorder such as dizziness, sweating and reduction in lacrimation, digestive apparatus symptom such as diarrhea and constipation, and organ disorder such as heart, kidney and eye. The present symptom is a disease of worse prognosis, which is developed in twenties to thirties, and is led to death after about ten years (Benson et al., "Trends in Neurosciences" 1989, vol.12, p.88-92).

Since TTR which is a causative protein for FAP is produced in mainly in liver, liver transplantation as therapy of FAP has become to be performed. Since progression of symptom of FAP is stopped by liver transplantation, and a part of autonomic symptom is recognized to be improved, it has been revealed that inhibition of production of atypical TTR in liver is an effective method for treating FAP. However, it is impossible from various reasons to apply liver transplantation to all patients. Moreover, since production of atypical TTR in a retina is not inhibited, there is a problem that ocular lesion progresses also after liver transplantation. Then, as therapy instead of this, it is thought indispensable to establish gene therapy where production of atypical TTR in liver and retina is inhibited.

On the other hand, by a human genome project, a whole gene sequence of a human has become to be read, it has been found out that there are many one base mutations between individuals and, further, it is being revealed that this one base mutation greatly influences on a morbidity of a disease and sensitivity of a drug. Therefore, in order to put gene therapy in practice, it is necessary to establish technique of correcting a particular specified one base mutation on a genome gene.

In 1996, an epoch-making method of mutating a specific base has been published by Kmiec et al. (Kmiec et al., "Science" 1996, vol.273, p.1386-1389). This method using a RNA-DNA chimeric oligonucleotide is a method of introducing an oligonucleotide which forms a double-stranded chain with a lesion of a gene desired to be mutated, into a cell, to cause homologous recombination with a genome gene, whereby, a genome gene is mutated. And, this method introduced an oligonucleotide into a lymphoblast cell to mutate a β-globin gene which is a causative gene for sickle cell anemia which is a gene disease. Since this report, it has been demonstrated that, using an oligonucleotide, gene mutation targeting a specific base in various cells can be performed. Further, in vivo, Kren et al. showed a possibility that, by mutating a gene of factor 9 in rat liver, hemophilia can be treated (Kren et al., "Nature Medicine" 1998, vol.4, p.285-290) and, subsequently, a possibility that the mutation can be used in treatment of Cligler-Najjar syndrome (Kren et al., "Proceeding of National Academy of Sciences USA" 1999, vol.96, p.10349-10354), and Duchenne-type muscular dystrophy was shown.

In addition, recently, it has been shown in an extract of a cell (Gamper et al., "Nucleic Acids Research" 2000, vol.28, p.4332-4339), or yeast (Saccharomyces cerevisiae) (Michael et al., "Nucleic Acids Reseach" 2001, vol.29, p.

4238-4250) that even a DNA oligomer like a RNA-DNA chimeric oligonucleotide can mutate a specific base of a genome gene, and utilization in gene therapy is expected.

However, it is common recognition to investigators in the art that a method of performing homologous recombination using a RNA-DNA chimeric oligonucleotide alone has no reproducibility, and that homologous recombination can not be performed at a high efficiency reported in these articles. In fact, Science journal which first published a paper of Kmiec et al., published a report that they performed investigation tracing the published article, and confirmed that the contents of the article are not reproduced. In addition, according to recent study of Kmiec et al. in the report, it has been made clear that a homologous recombination efficacy when a RNA-DNA chimeric oligonucleotide is used alone is 0.0002% to 0.005%, and a homologous recombination efficiency when a DNA oligomer is used alone is about 0.016% to 0.02% (Taubes, "Science" 2002, vol.298, p.2116-2120). Therefore, there is demanded development of a promotion system in which homologous recombination is performed effectively using these oligonucleotides.

On the other hand, the greatest problem for clinically putting gene therapy using an oligonucleotide in practice is a method of delivering an oligonucleotide into a cell in a living body. In fact, in all studies which have been previously performed, a method of introducing an oligonucleotide into a cell using a delivering technique such as electroporation, gene gun, liposome, and polycation is problematic on toxicity and convenience, and none of them have been subjected to clinical study. In particular, it is the fact well known to researchers in the art that many liposomes and cationic polymers are difficult to be stored in a long term in the state where mixed with an oligomer and, moreover, an introduction efficiency greatly depends on skill at preparation. Therefore, there is demanded development of a delivering system which can introduce an oligonucleotide into a cell safely and effectively to convert a genome gene and, at the same time, is safe and highly convenient.

In a method of delivering an antisense oligonucleotide, since a messenger RNA as a target is mainly present in a cytoplasm, it has not been demanded that an oligonucleotide is localized in a specified site in a cell. On the other hand, in order to perform conversion of a genome gene, it is necessary to introduce and localize an oligonucleotide into a nucleus to enhance an efficacy of gene conversion, and there is desired development of a delivering system by which an oligonucleotide is localized in a nucleus of a cell effectively.

DISCLOSURE OF THE INVENTION

An object of the present invention is to provide a preparation which promotes localization of an oligonucleotide in a nucleus by effectively introducing the oligonucleotide into a cell, a preparation which promotes conversion of a nucleotide sequence of a desired genome gene, and a preparation for gene therapy. The present inventors have been engaged in study regarding sideration of, pathology of, and a treatment method of a patient of familial amyloidotic polyneuropathy (FAP) for many years, and we intensively studied gene therapy of FAP type I (FAP ATTR Val30 Met) in which 30th valine of TTR is mutated into methionine due to point mutation of a transthyretin (TTR) gene and, as a result, found out a preparation which is effective in not only treatment of various gene diseases including FAP, but also in conversion of a site-specific gene in vitro by satisfying the following requirements, which resulted in completion of the present invention.

That is, the gist of the present invention is as follows:

[1] A preparation for facilitating site-specific gene conversion, comprising at least collagen and an oligonucleotide for gene conversion;

[2] A preparation for site-specific gene therapy, comprising at least collagen and an oligonucleotide for gene conversion.

The collagen is preferably water-soluble collagen, and the water-soluble collagen is preferably atelocollagen.

It is preferable that the oligonucleotide for gene conversion is an oligonucleotide consisting of at least 20 bases, specifically, it is preferable that the oligonucleotide is a RNA/DNA chimeric oligonucleotide or a DNA oligonucleotide.

It is preferable that the oligonucleotide for gene conversion is an oligonucleotide having a nucleotide sequence which forms a Watson-Crick-type base pair containing mismatch pairing of 1 to 3 bases with a sense strand or an antisense strand of a gene to be converted, or the oligonucleotide for gene conversion is an oligonucleotide having a nucleotide sequence which forms a Watson-Crick-type base pair containing deletion or insertion of 1 to 3 bases with a sense strand or an antisense strand of a gene to be converted.

It is preferable that the mismatch pairing is located at a central part of an oligonucleotide, or the deletion or insertion of bases is located at a central part of an oligonucleotide.

It is preferable that the preparation for facilitating or the therapy has a dosage form of a solution, and it is preferable that the preparation contains a phosphate salt in a range of 0.01M to 0.1M, and contains a sodium salt in a range of 0.07M to 0.14M.

In addition, it is preferable that the preparation for facilitating or the therapy has a dosage form of a solid.

It is preferable that an oligonucleotide for gene conversion and a collagen are a particulate associated body, and a long diameter of a particulate associated body is 300nm to 50μm.

It is preferable that the preparation for facilitating or the therapy which is solution-like contains collagen at 0.01 to 1.0% by weight, or contains collagen in a range of 0.01 to 0.25% by weight.

[3] A preparation for facilitating site-specific gene conversion or a preparation for gene therapy obtained by dissolving collagen in a solution containing 0.01M to 0.1M of a phosphate salt and 0.07M to 0.14M of a sodium salt, adding an oligonucleotide solution for gene conversion containing the same concentration of a phosphate salt and the same concentration of a sodium salt thereto, and stirring this under a temperature of 1 to 10°C.

[4] A method of arbitrarily converting a specific base on a genome gene in a nucleus of a cell, comprising contacting the preparation for facilitating gene conversion with the cell.

It is preferable that the cell is a mammal cell, a yeast or a fungus.

[5] A preparation for facilitating for localizing an oligonucleotide localization in a nucleus, comprising at least a collagen and an oligonucleotide.

It is preferable that the preparation for facilitating intranuclear localization contains a phosphate salt in a range of 0.01M to 0.1M, and contains a sodium salt in a range of 0.07M to 0.14M.

It is preferable that the oligonucleotide and collagen are a particulate associated body, and a long diameter of the particulate associated body is 300nm to 50μm.

It is preferable that the preparation for facilitating intranuclear localization contains collagen in a range of 0.01 to 1.0% by weight, or contains collagen in a range of 0.05 to 0.25% by weight.

[6] A method of gene conversion of a cell, comprising contacting a composition containing at least a collagen and an oligonucleotide for gene conversion with a cell in a living body by oral, nasal, via lung, intraportal, intramuscular, subcutaneous, organ surface, intraorganic or transdermal administration.

[7] A method of treating a gene disease using the method as defined in [6].

BRIEF DESCRIPTION OF THE DRAWINGS

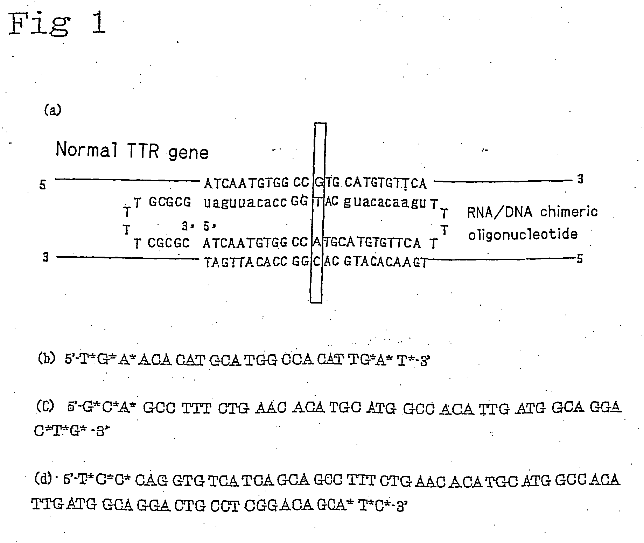

Fig.1 shows a structure of a RNA/DNA chimeric oligonucleotide and a DNA oligonucleotide.

(a) RNA/DNA chimeric oligonucleotide, (b) 25mer DNA oligonucleotide, (c) 45mer DNA oligonucleotide, (d) 74mer DNA oligonucleotide. A RNA part (small letter) of a RNA/DNA chimeric oligonucleotide becomes 2'-O-methyl-RNA, and 3 bases (*) from both ends of a DNA oligonucleotide becomes phosphorothioate, preventing degradation.

Fig.2 is micrographs (a) and (b) showing uptake of an atelocollagen-embedded DNA oligonucleotide into HepG2 cells and, for comparison, a micrograph (c) showing results in a HVJ-liposome-encapsulated DNA oligonucleotide. Magnification is 100-fold in all cases.

Fig.3 is a micrograph showing nature of an atelocollagen-embedded DNA oligonucleotide.

(a): Only DNA oligonucleotide.
(b): 0.05% atelocollagen-embedded DNA oligonucleotide 74mer.

Fig.4 is a graph showing results of mass spectroscopy of transthyretin in a transgenic mouse serum exhibiting production of normal transthyretin due to an atelocollagen-embedded DNA oligonucleotide.

A: Transthyretin extracted from serum of a non-treated transgenic mouse.
B: Transthyretin extracted from serum of a transgenic mouse to which an atelocollagen-embedded DNA oligonucleotide has been administered.

BEST MODE FOR CARRYING OUT THE INVENTION

A first aspect of the present invention relates to a preparation for facilitating site-specific gene conversion, comprising at least a collagen and an oligonucleotide for gene conversion.

In the present invention, "collagen" means all "collagens" which are usually used in medical, cosmetic, industrial and food fields. It is preferable to use water-soluble or solubilized collagen. The water-soluble collagen is soluble in acidic or neutral water, or a salt solution, and the solubilized collagen includes an enzymatically solubilized collagen which is solubilized with an enzyme, an acid soluble collagen which is solubilized with an acid, and an alkali soluble collagen which is insolubilized with an alkali, and it is preferable that all can pass through a membrane filter having a pore size of 1 micrometer. Water-solubility of collagen depends on a crosslinking degree of collagen. Since as a crosslinking degree is higher, collagen is solubilized, a crosslinking degree of collagen used in the present invention is preferably a tri- or less-mer, more preferably di- or less-mer. A molecular weight of collagen is preferably about 300 to about 900 thousands, more preferably about 300 to about 600 thousands. Collagen which has been extracted from any animal species may be used, and collagen extracted preferably from a vertebrate, collagen extracted further preferably from a mammal, birds or fishes, collagen extracted from more preferably from a mammal, or birds having a high denaturation temperature. Any type of collagen may be used, and types I to V are preferable from a viewpoint of an amount of existence in an animal body. Specifically, examples include type I collagen which was extracted from a mammal dermis with an acid, more preferably type I collagen which was extracted from a calf dermis with an acid, and type I and type III collagens produced by genetic engineering, and the like. In addition, from a viewpoint of safety, atelocollagen from which a telopeptide having high antigenicity has been enzymatically removed, or genetically produced atelocollagen is desirable. Alternatively, collagen having a side chain which has been modified if necessary, and crosslinked collagen can be used. Examples of collagen having a modified side chain include succinylated or methylated collagen. Examples of crosslinked collagen include collagen treated with glutaraldehyde, hexamethylene diisocyanate or polyepoxy compound (Fragrance Journal 1989-12, 104-109; Japanese Patent Publication No. 7-59522).

The collagen may be mixed with other biocompatible material. Examples of the biocompatible material include gelatin, fibrin, albumin, hyaluronic acid, heparin, chondroidin sulfate, chitin, chitosan, alginic acid, pectin, agarose, hydroxyapatite, polypropylene, polyethylene, polydimethylsiloxane, and a polymer of glycolic acid, lactic acid or amino acid and a copolymer thereof, and a mixture of two or more kinds of these biocompatible materials.

The "oligonucleotide for gene conversion" in the present invention is a single-stranded nucleotide having such a length and nucleotide sequence that a genome gene is converted. A length of the oligonucleotide is preferably at least 20 bases, more preferably 25 to 100 bases, further preferably 30 to 75 bases.

From a viewpoint of efficiency of gene conversion, the oligonucleotide is preferably a RNA/DNA chimeric oligonucleotide or a DNA oligonucleotide. From a viewpoint of easiness of synthesis and purification, a DNA oligonucleotide is more preferable.

In order to enhance stability to a nuclease in a living body or a cell, the oligonucleotide may have one or more nucleic acid analogues in a molecule. The nucleic acid analogue is an analogue designed for the purpose of inhibiting degradation of a DNA strand or a RNA strand with an enzyme. Examples include phosphorothioate in which an oxygen atom of a phosphate diester linkage site is substituted with one sulfur, methyl phosphate in which the oxygen atom is substituted with a methyl group, phosphoroamidate in which the oxygen atom is substituted with an amine group, phosphorodithioate in which two oxygen atoms at a phosphate diester linkage site are substituted with sulfurs, methyl phosphorothioate in which the two oxygen atoms are substituted with one sulfur atom and a methyl group, and 2'-O-methyl RNA, 2'-O-methoxyethyl RNA and Locked nucleic acid (trade name) (LNA) in which a sugar part is chemically modified (Bioclinica, 12, 166-170, 1997, Biochemistry, 41, 4503-4510,2002). When the number of nucleic acid analogues contained in an oligonucleotide is expressed regarding a phosphorothioate-type nucleic acid analogue as a representative, the number of phosphorothioate bond is preferably around 4 to 6. It is desirable that a phosphorothioate bond is introduced on both sides of a position at least 3 bases or more apart from a base to be mutated. When a position approaches a base to be mutated, there is a tendency that a conversion efficiency of a gene is rather reduced. More preferably, it is desirable that the bond is present over consecutive 2 or more bases at both end sites of an oligonucleotide. The oligonucleotide may be such that a base part is chemically modified.

It is preferable that the oligonucleotide is designed to be an oligonucleotide containing a nucleotide sequence forming a Watson·Crick-type base pair containing mismatch pairing of 1 to 3 base pairs at an approximately central part, with a sense strand or an antisense strand of a gene to be mutated.

That is, it is preferable to design an oligonucleotide in which a nucleotide sequence of 20 or more bases of a genome gene to be converted is selected, a nucleotide sequence of 1 to 3 bases located in the interior of the sequence is substituted with a desired nucleotide sequence, and a remaining nucleotide sequence is designed to be a complementary sequence forming a Watson·Crick-type base pair (i.e. double-stranded). The complementary sequence may be to a sense strand or an antisense strand of a genome gene, preferably to a sense strand. When such the oligonucleotide and genome gene form a Watoson·Click-type base pair, mismatch paring of 1 to 3 base pairs is contained in the base pairing. In order to enhance an efficiency of gene conversion, it is preferably that the mismatch pairing is located at a central part of an oligonucleotide.

When such the oligonucleotide for gene conversion is used, mutation of 1 to 3 bases in a genome gene can be site-specifically repaired and, conversely, mutation of 1 to 3 bases can be site-specifically introduced in a genome gene. When the mutation is 2 bases or 3 bases, the mutation may be continuous, or discontinuous.

It is preferable to design the oligonucleotide for gene conversion so as to be an oligonucleotide containing a base sequence forming a Watson·Crick-type base pair containing deletion or insertion of 1 to 3 bases, with a sense strand or an antisense strand of a gene to be converted.

That is, it is preferable to design an oligonucleotide in which a nucleotide sequence of 20 or more bases of a genome gene to be converted is selected, a nucleotide sequence of 1 to 3 bases located in the interior of the sequence is deleted, or a nucleotide sequence of 1 to 3 bases is inserted into the interior of the sequence, and a remaining nucleotide sequence is designed to be a complementary sequence forming a Watson-Crick-type base pair (i.e. double stranded). The complementary sequence may be to a sense strand or an antisense strand of a genome gene, preferably to a sense strand. When such the oligonucleotide and genome gene form a Watson-Crick-type base pair is formed, a loop of 1 to 3 bases is contained in the base paring. In order to enhance an efficiency of gene conversion, it is preferable that the loop is located at a central part of an oligonucleotide.

When such the oligonucleotide for gene conversion is used, mutation of 1 to 3 bases in a genome gene can be site-specifically deleted and, conversely, mutation of 1 to 3 bases can be site-specifically inserted into a genome gene. When the mutation is 2 bases or 3 bases, the mutation may be continuous, or discontinuous.

Design of a RNA/DNA chimeric oligonucleotide is such that, in addition to the aforementioned conditions, for example, as shown in Fig.1 (a), a nucleotide sequence in which two kinds of nucleotide sequence parts being capable of forming a Watson-Crick-type base pair with a sense strand and an antisense strand, respectively, and an arbitrary intervening sequence part not forming a base pair are consecutive, can be selected. A method of designing a RNA/ DNA chimeric oligonucleotide is disclosed, for example, in US5,731,181, and US5,756,325.

A second aspect of the present invention relates to a preparation for site-specific gene therapy comprising at least a collagen and an oligonucleotide for gene conversion.

A collagen and an oligonucleotide for gene conversion contained in the preparation for gene therapy of the present invention are the same as the collagen and the oligonucleotide for gene conversion contained in the preparation for facilitating gene conversion.

A dosage form of the preparation for facilitating site-specific gene conversion and the preparation for gene therapy of the present invention (hereinafter, also referred to as present preparation) may be any of solution-like, suspension-like, gel-like, film-like, and solid-like (rod-like, powdery) and the like, and is selected depending on utility. For example, when gene conversion of a substrate adhesive cell is performed using the preparation for facilitating site-specific gene conversion of the present invention, it is desirable to use a film-like or powdery preparation fixed to a substrate surface in addition to a method of adding a preparation in the solution state or the suspension state to a culture of a cell, in order to enhance contact of an oligonucleotide with a cell. When gene conversion of a suspended cell is performed, it is preferable to use a preparation in the solution state or the suspension state. Importance of dosage form selection becomes further great when gene therapy is performed by administering the preparation for gene therapy of the present invention to a living body. When therapy targeting a cell of a whole body is performed, a preparation is desirably solution-like or suspension-like so that the preparation can be intravascularly administered. However, generally, in the field of gene therapy, in order to reduce a possibility of manifestation of unexpected side effect as much as possible, it is thought that, even if an oligonucleotide for not performing gene conversion in a normal cell, introduction of an oligonucleotide into a cell requiring no gene conversion is not preferable. When gene conversion is intended to be performed on only a cell at a limited site, it is desirable to maintain a local concentration of a preparation high, and use a gel-like, film-like or solid-like preparation which can inhibit diffusion of a preparation to the surrounding. In addition, in the case where it is necessary to remove a preparation for gene therapy when desired gene conversion is performed by treating a patient cell with the preparation for gene therapy of the present invention in vitro, and a gene-converted cell is transplanted into a patient, a film-like or solid-like dosage form is desirable from a viewpoint of easy removal.

A process for preparing these dosage forms is described in International Publication 01/97857 pamphlet (title of the invention: preparation for introducing oligonucleotide) and, in the present invention, any process may be used.

A preferable aspect of the solution-like, suspension-like or gel-like preparation of the present invention will be explained below.

Mechanism that an oligonucleotide in the preparation of the present invention causes gene mutation is thought to be due to homologous recombination of an oligonucleotide and a gene, or mismatch repair by formation of a hybrid of an oligonucleotide and a gene, but it is not clear which is right. Regardless of any mechanism, it is necessary that an oligonucleotide and a gene form a hybrid at a part targeted by the oligonucleotide. Usually, when a cell is not in a cell division phase, and does not produce a protein, since a gene forms a stable double-stranded chain, and interacts with histone, whereby, a gene is condensed at a high density and is present in a nucleus, it can not be expected that a foreign oligonucleotide dissociates a double-strand chain of a gene, and forms a hybrid with a target gene chain. Therefore, in order that an oligonucleotide in the preparation of the present invention forms a hybrid with a target gene chain, and performs desired gene conversion, it is necessary that a double-stranded chain of a gene is dissociated for replication of a gene accompanied with cell division, or transcription of a gene accompanied with protein production, in a term during which an oligonucleotide is present in a nucleus. Generally, since it is known that, when a cell undergoes damage due to extrinsic factor, protein production ability and cell division ability of a cell are remarkably reduced, it is desirable that the preparation of the present invention is formulated and designed so that damage is not given to a cell with which the preparation is contacted, and an oligonuleotide is introduced. That is, it is desirable that the solution-like, suspension-like, or gel-like preparation of the present invention is isotonic with a cell. When the preparation of the present invention contains a phosphate salt and a sodium salt, it is desirable that they are contained in a range of phosphate salt 0.1 M to phosphate salt 0.01 M and sodium salt 0.14 M, and it is more preferable that they are contained in a range of phosphate 0.05 M and sodium salt 0.07 M to phosphate salt 0.01 M and sodium salt 0.14 M.

Further, it is preferable to prepare the preparation of the present invention so that an oligonucleotide for gene conversion and collagen become a particulate associated body, in order to enhance a gene conversion efficiency. Herein, the "associated body" means that a complex in which collagen with many positive charges and an oligonucleotide with negative charges are attracted electrically in a molecule, is associated with other collagen. Formation of this associated body is such that a pillar collagen molecule having a long diameter of about 300nm and a diameter of about 1.5nm is mainly associated parallel with a long axis direction of a molecule, and an associated body is mainly extended in a long axis direction of a molecule. Therefore, an associated body can take various shapes such as fiber, fine fiber and particle depending on a degree of extension. Among them, an associated body in the present invention is preferably particulate from a viewpoint of an efficiency of tranferability of an oligonucleotide into a cell, in particular, into a nucleus.

A long diameter of the particulate associated body is preferably 300nm to 50μm, more preferably 300nm to 30μm.

In order to form a particulate associated body, a concentration and a ratio of a collagen and an oligonucleotide to be mixed, a salt concentration, temperature, and pH are adjusted.

When a salt is not present, since a long diameter of an associated body depends on collagen concentration at mixing, in order to obtain an associated body having the aforementioned preferable long diameter, it is desirable that a collagen concentration at mixing is 1.0 to 0.005 % by weight, more preferably 0.5 to 0.005 % by weight, further preferably 0.05 to 0.005 % by weight. And, as a collagen concentration is reduced, an oligonucleotide concentration at which an associated body is formed is reduced. As a method of obtaining a more preferable associated body at high concentration, there is a method of dissolving collagen in a solution containing 0.01 M to 0.1M of a phosphate salt and 0.07M to 0.1M of a sodium salt in advance, to form a fine collagen fiber or a collagen molecule associated body and, occasionally, maintaining a collagen molecule in the single molecule state, and adding an oligonucleotide thereto to form an associated body with a collagen fiber, a collagen molecule associated body or a collagen single molecule. In addition, since a temperature influences on formation of a collagen fiber, in order to obtain an associated body having the aforementioned preferable long diameter, a temperature at mixing is desirably 1 to 10°C, more preferably 1 to 5°C.

Therefore, the solution-like preparation of the present invention is obtained by dissolving collagen in a solution containing 0.01 M to 0.1 M of a phosphate salt and 0.07M to 0.14M of a sodium salt, adding a solution of an oligonucleotide for gene conversion containing the same concentration of a phosphate salt and the same concentration of a sodium salt thereto, and stirring this under a temperature of 1 to 10°C.

A collagen concentration at mixing is usually 50μg/ml to 10mg/ml, and an oligonucleotide concentration at mixing is usually 20μg/ml to 1mg/ml.

A ratio of the number of collagen molecules and the number of nucleotide monomers of an oligonucleotide which formed an associated body is 1:1 to 1:200, preferably 1:3 to 1:150, more preferably 1:3 to 1:120.

A pH of a solution at mixing is pH 5 to 9, preferably pH 6 to 8.

By preparing the preparation of the present invention under such the conditions, a solution-like preparation containing a particulate associated body can be provided.

When used mainly in gene conversion or intranuclear localization of an oligonucleotide in vitro, a concentration of collagen in the solution-like preparation is preferably in a range of 0.01 to 1.0% by weight.

When used mainly in gene conversion, gene therapy or intranuclear localization of a oligonucleotide in vivo, a concentration of collagen in the solution-like preparation is preferably in a range of 0.01 to 0.25% by weight.

Further, the solution-like preparation of the present invention can contain 0.01 to 1% by weight of EDTA for stabilizing an oligonucleotide, and 0.01 to 1% by weight of a surfactant for preventing adhesion onto a container and an administration equipment.

A preferable aspect of the film-like, or solid-like (rod-like, powdery) preparation of the present invention will be explained below.

The film-like or solid-like preparation is obtained by concentrating and drying the aforementioned solution-like preparation. That is, the solution-like preparation is cast on a planar plate, and dried at a temperature of 40°C or lower, whereby, a film-like preparation can be prepared. Alternatively, a solution-like preparation is centrifuged to precipitate an associated body of an oligonucleotide and a collagen, and precipitates are dried at 40°C or lower, whereby, a powdery preparation can be prepared. A rod-like preparation can be prepared by a method of lyophilizing the thus obtained powdery preparation or solution-like preparation to obtain a sponge-like compound, and compressing the sponge-like compound to prepare a rod-like preparation, or by a method of adding a small amount of water to a powdery preparation and a sponge-like preparation, and kneading them to obtain a solution having a high concentration, extruding this through a nozzle, and drying it at 40°C or lower to obtain a rod-like preparation.

For the purpose of maintaining a shape of a preparation, a film-like or a solid-like preparation can contain a pharmaceutically acceptable additive such as albumin, gelatin, chondroitin sulfate, agarose, sorbitol and sucrose in a range of 10 to 80% by weight of a whole preparation in addition to an additive contained in a solution-like preparation.

A particle diameter of a powdery preparation can take various shapes depending on an excipient, and a long diameter of an associating body to be formed of contained oligonucleotide and collagen is preferably 300 nm to 50μm, more preferably 300 nm to 30μm.

In a rod-like solid-like preparation, it is desirable that a diameter is 0.1mm to 2.0mm, and a length is 3mm to 20mm, and it is more desirable that a diameter is 0.3mm to 1.0mm, and a length is 3mm to 10mm, so that it can be administered locally by injection.

An amount of an oligonucleotide contained in a solid preparation is usually 10μg to 100μg per 1mg of a solid preparation, and an amount of collagen is usually 990μg to 250μg per 1mg of a solid preparation.

A third aspect of the present invention relates to a method of arbitrarily converting a specific base on a genome gene in a nucleus of a cell. That is, the conversion method of the present invention is characterized in that, by contacting the preparation for facilitating site-specific gene conversion of the present invention with a cell, an oligonucleotide contained in the preparation for facilitating is transferred and localized in a nucleus of a contacted cell, thereby, conversion of a desired base is performed.

A cell to be converted is not particularly limited as far as it is an eukaryote, and examples include a yeast, a fungus, a plant cell and an animal cell, preferably, a mammal cell, a yeast and a fungus.

Whether a specific base has been converted or not can be investigated by contacting with the preparation for facilitating gene conversion of the present invention, recovering a cell after a constant term, and amplifying a gene region containing a specific base by a PCR method or the like.

The preparation for gene therapy of the present invention can be used in treatment of various gene diseases. Examples of diseases to be treated include diseases caused by that a normal protein is not expressed due to point mutation of a gene (including mutation of 1 to 3 bases), deletion mutation or insertion mutation (including mutation of 1 to 3 bases). Examples of such the diseases include familial amyloidotic polyneuropathy (FAP), Fabry's diseases, Wilson's diseases, thalassemia, sicklemia, myodystrophy, cystic fibrosis, factor 5 Leyden's abnormality, and biotin-dependent multiple carboxylase deficiency.

In the case of FAP, representative examples include a disease due to atypical transthyretin (TTR) in which 30th valine of TTR is mutated into methionine by point mutation (I type FAP), and a disease due to atypical TTR in which 84th isoleucine of TTR is mutated into serine (II type FAP). In addition, regarding FAP, FAPs due to more than 90 kinds of various TTR point mutations have been previously reported, and the present gene therapy can be applied to all of these types of FAPs. Since TTR is mainly produced in liver, the gene therapy of the present invention can be administered to a liver cell as a target. In addition, amyloid sedimentation due to atypical TTR also causes visual disorder accompanied with whitening of a vitreous body of eyes, and this disorder can be treated by administering the gene therapy of the present invention directly to a retina of eyes.

The preparation for gene therapy of the present invention can be administered transdermally, subcutaneously, intradermally, nasally, via lung, intramuscularly, intracerebrally, tissularly (organ surface, intraorgan), intravascularly (intravenous, intraportal) or orally depending on the therapeutic purpose.

A dose of the preparation for gene therapy of the present invention can be easily adjusted by a solution amount in the case of a solution-like preparation, by an area in the case of a film-like preparation, by a diameter and a length in the case of a rod-like preparation, and a powder volume or weight in the case of a powdery preparation.

An optimal dose of the preparation for gene therapy of the present invention is different depending on an application disease, an administration part, an administration method, a kind of a dosage form, and a gender, an age and symptom of a patient, and an amount of an oligonucleotide in a preparation is for example 0.001mg/kg to 40mg/kg, preferably 0.01mg/kg to 30mg/kg patient.

An oligonucleotide in the preparation for gene therapy after administration can effectively convert mutation in a genome gene, that is, can repair mutation. In the case of FAP, as a result of gene repair, normal TTR is produced, and an amount of atypical TTR is reduced to inhibit formation of amyloid, whereby, symptom of FAP is improved.

A fourth aspect of the present invention provides a preparation for facilitating an oligonucleotide localization in a nucleus, comprising at least a collagen and an oligonucleotide. In the present preparation, although an oligonulceotide designed in conformity with conditions of the oligonucleotide for gene conversion can be used, an arbitrary oligonucleotide having a length normal to a oligonulceotide can be used preferably.

The present preparation can take various dosage forms like the preparation for facilitating gene conversion, and a solution-like dosage form is preferable. Concentrations of a phosphate salt and a sodium salt contained in the solution-like present preparation are the same as those described above. Other conditions (a concentration of collagen, a ratio of the number of collagen molecules to the number of oligonucleotide monomers. a pH and a temperature of a solution at mixing) are the same as those described above.

By contacting the present preparation with a cell, an oligonucleotide contained in the preparation can be effectively localized in a nucleus of a cell. Whether an oligonucleotide has been localized in a nucleus of a cell or not can be confirmed by labeling the oligonucleotide with a fluorescent pigment, and observing this with a fluorescent microscope.

Examples

The present invention will be specifically explained by way of Examples below, but the present invention is not limited by these Examples at all. In Examples, % denoting a collagen concentration means % by weight.

Preparation Example 1

Preparation for facilitating site-specific gene conversion

A preparation for site-specific conversion of a gene of TTR associated with FAP and a preparation for site-specific conversion of a gene of α-glactosidase associated with Fabry's disease which were used in the following Experimental Examples and Examples were prepared. Equivalent amounts of 3.83 to 50μM oligonucleotide having a nucleotide sequence described in any of SEQ ID NOs: 2 to 12 and 10μg/ml to 1g/ml of atelocollagen were mixed in a 10mM phosphate buffer (pH 7.0) containing 0.14M sodium chloride at 2°C, to prepare a solution preparation containing an associated body of an oligonucleotide and collagen (DNA oligonucleotide embedded in atelocollagen).

Example 1

Gene conversion rate in HepG2 cell

1) Oligonucleotide for gene conversion
What is seen most frequently in FAP is FAP type I (FAP ATTR Val30Met) in which 30th valine of TTR is mutated into methionine. Then, in order to convert a normal TTR gene of a HepG2 cell having a normal TTR gene so as to express ATTR Val30Met, DNA oligonucleotides shown in Figs. 1 (b) to (d) were designed. In order to study an optimal length of a DNA oligonucleotide, three kinds of 25mer (SEQ ID NO:2), 45mer (SEQ ID NO: 3) and 74mer (SEQ ID NO:4) were synthesized. The oligonucleotides were designed based on a human TTR gene, and 74mers synthesized by design based on mouse and rabbit TTR genes are descried in SEQ ID NO:5 and SEQ ID NO:6.
2) Transfection method
In order to study an efficiency of introduction of a gene into a HepG2 cell, a 5'-terminus of an oligonucleotide to be introduced was labeled with FITC. Using Fugene6 (manufactured by Roche), ExGen 500 (manufactured by MBI Fermentas), a HVJ-liposome (gifted from Dr. Yasushi Kaneda, Osaka University Graduate School of Medicine, Gene Therapeutics) or an atelocollagen preparation (containing an oligonucleotide labeled with FITC) prepared in Preparation Example 1, optimization of a transfection method was studied.
3) Study of gene conversion rate in HepG2 cell by DNA oligonucleotide
On previous day, $2 \times 10^5$ HepG2 cells (purchased from Dainippon Pharmaceutical Co., Ltd.) were seeded on a 12-well plate, this was transfected with 300μl and 600μl of a DNA oligonucleotide 74mer (3.83μM) embedded in 0.1% atelocollagen, and cells were recovered after 5 days (at 300μl addition) or 6 days (at 600μl addition). A DNA was extracted from the recovered cells, and a gene conversion rate was calculated using a MASA method (mutant allele specific amplification) and real time PCR designed so as to effectively amplify only an abnormal allele (ATTR Val30Met), employing a mutated DNA-specific oligonucleotide designed so that a base corresponding to a mutated sequence became a 3'-terminus.
4) Study of nature of oligonucleotide embedded in atelocollagen
An associated body of 8μl of atelocollagen and an oligonucleotide (prepared in Preparation Example 1) was mixed with 2μl of a 5-fold diluted single-stranded nucleic acid staining fluorescent reagent YOYO (Molecular Probe) which stains a single-stranded DNA, and this was observed under a fluorescent microscope.
5) Study of optimal conditions for gene conversion in HepG2 cell by DNA oligonucleotide
On previous day, $2 \times 10^5$ HepG2 cells were seeded on a 12-well plate, and transfected with each 600μl (culture solution 600μl) of a DNA oligonucleotide (10μM 25mer, 10μM 45mer, 10μM 74mer) embedded in 0.1% atelocollagen, or a DNA oligonucleotide (50μM 25mer, 25μM 45mer, 10μM 74mer) embedded in 0.5% atelocollagen, and cells were recovered after 5 days. A DNA was extracted from the recovered cells, and a gene conversion rate was calculated using a MASA method and real time PCR.

Based on the above experimental results, a gene conversion efficiency was confirmed.

(A) Rate of localization of oligonucleotide in cellular nucleus

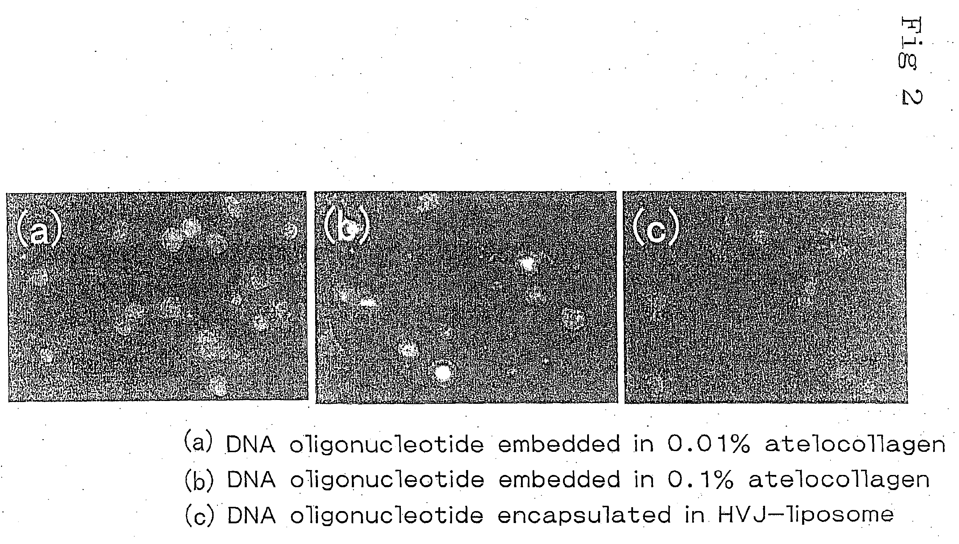

As shown in Figs. 2 (a) and (b), it is seen that a rate of localization of a DNA oligonucleotide in a nucleus of HepG2 cells is such that, in Fugene6, ExGen500, HVJ-liposome and atelocollagen preparations, about 50% of a DNA oligonucleotide embedded in atelocollagen was incorporated into HepG2 cells, and a further introduced DNA oligonucleotide is localized in a nucleus. In all other methods, weaker fluorescent light than this was shown, and a rate of localization in a nucleus was low.

(B) Gene conversion rate in HepG2 cell by DNA oligonucleotide

In the previous reports, in an experiment using a DNA oligonucleotide, in order to investigate an optimal length of a DNA oligonucleotide, an efficiency of gene repair was compared and studied using 25mer to 74mer DNA oligonucleotides, and it is known that 45mer and 74mer DNA oligonucleotides have a relatively high gene repair rate. Then, a gene conversion rate in HepG2 cells was investigated using a DNA oligonucleotide 74mer (SEQ ID NO: 4) embedded in atelocollagen, under the condition of 3), when 300µl or 600µl of a DNA oligonucleotide (oligonucleotide concentration: 3.83µM) embedded in atelocollagen was added, a gene conversion rate was 0.5% and 1% or smaller, respectively, while under the condition of 5) (oligonucleotide concentration: 10µM), by increasing an atelocollagen concentration in a preparation from 0.1% to 0.5%, and adding 600µl of a DNA oligonucleotide embedded in atelocollagen to 600µl of a cell culture, a gene conversion rate was increased from 1% to 10%. It is thought that a gene conversion rate using the present preparation is increased to a constant level in a dose-dependent manner. In a DNA oligonucleotide 25mer (SEQ ID NO: 2), a gene conversion rate was 0% at an atelocollagen concentration of 0.1% (DNA oligonucleotide concentration: 10µM), and about 0.5% at 0.5% (DNA oligonucleotide concentration: 50µM) and, in 45mer SEQ ID NO: 3), a gene conversion rate was 0% at an atelocollagen concentration of 0.1%. (DNA oligonucleotide concentration: 10µM), and about 1% at 0.5% (DNA oligonucleotide concentration : 25µM).

These results show that a chain length of a DNA oligonucleotide used in the present invention is desirably 45mer or longer, more desirably 74mer or longer.

(C) Study of nature of DNA oligonucleotide embedded in atelocollagen

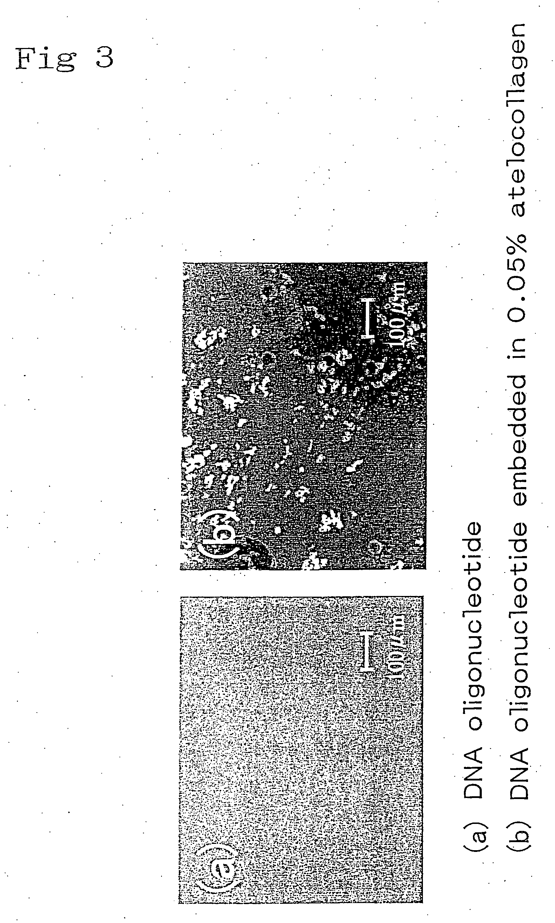

Results of observation with a fluorescent microscope of the above 4) are shown in Fig. 3. From Fig. 3, no particle is observed in a DNA oligonucleotide alone, while an associated body particle was observed, and an average long diameter of an associated body particle was 18.73µm in a DNA oligonucleotide (SEQ ID NO: 5) embedded in 0.05% atelocollagen.

Example 2

Study of gene conversion in eyes in home rabbit by DNA oligonucleotide

Camera anterior bulbi liquid of home rabbit was removed, a DNA oligonucleotide (10µM 74mer, SEQ ID NO: 6) embedded in 0.5% atelocollagen or a DNA oligonucleotide (30µM 74mer) embedded in 1% atelocollagen was directly injected into a vitreous body (left eye), or injected in a vitreous body (right eye) after excision of a vitreous body. After one month, eyes were isolated, a RNA was extracted, and a first strand cDNA was synthesized using a reverse transcriptase. Using this cDNA as a template, and employing a MASA method and real time PCR, a copy number of normal TTR and ATTR Val30mET was determined to calculate a gene conversion rate.

A gene conversion rate was calculated by the following fomula:

copy number of ATTR Val30Met / (copy number of ATTR Val30Met +

copy number of normal TTR gene) $\times$ 100(%).

A gene conversion rate was higher in a 74mer DNA oligonucleotide embedded in 1% atelocollagen than in a 74mer DNA oligonucleotide embedded in 0.5% atelocollagen. In addition, excision of a vitreous body provided a higher gene conversion rate (about 1%).

Example 3

Study of gene conversion in mouse liver

The following preparations 1 to 3 were administered to a heterotransgenic mouse having normal and abnormal mouse transthyretin (ATTR Val30Met) genes and a homotransgenic mouse having an abnormal mouse transthyretin gene (Analysis of Genetic Amyloidosis Sideration Mechanism using Mutation-Introduced Mouse, Shuichiro Maeda et al., Welfare Science Research Fee Subsidy Specified Disease Strategy Research Undertaking "Study regarding Amyloidosis", Year Heisei 13, Comprehensive Study Report, p39-41), and a gene conversion rate was investigated.

Oligonucleotide: 74mer (SEQ ID NO: 5) in which a base to be gene-converted is disposed at a center, and 3 bases at both ends are made to be a phosphorothioate oligonucleotide
Preparations:

Preparation 1:    oligonucleotide 10μM, atelocollagen 0.5%
Preparation 2:    oligonucleotide 10μM, atelocollagen 0.2%
Preparation 3:    oligonucleotide 10μM, atelocollagen 0.05%

Administration method: Each 0.2ml of the preparations was directly administered to two places of one liver lobe of mouse liver (total 0.4ml).

After reared for 3 weeks, a liver lobe to which the preparation had been administered, and a liver lobe to which no preparation had been administered were taken, a gene was extracted, and a ratio of a normal gene in all transthyretin genes was measured regarding both liver lobes using a MASA method and real time PCR.

In addition, after reared for 3 weeks, a blood was taken from an untreated homotransgenic mouse (mouse transthyretin gene ATTR Val30Met) and a homotransgenic mouse to which the preparation had administered, an anti-transthyretin antibody was added to serum to perform immunological settlement, extracted transthyretin was analyzed using a mass spectroscopic apparatus (matrix-assisted laser diffraction ionization/time-of-flight mass spectrometry), and whether normal transthyretin was produced or not was studied.

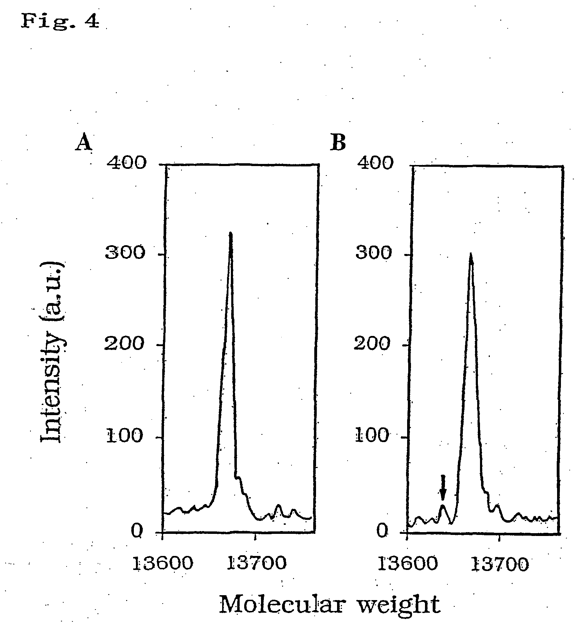

Results: a ratio of a normal gene in both liver lobes of a preparation 3-administered heterotransgenic mouse was 60.7% in a liver lobe to which the preparation had been administered, and 51% in a liver lobe to which no preparation had been administered. Therefore, it is thought that 10% gene repair effect was obtained. A preparation 2-administered mouse died during rearing (cause is unclear). In a preparation 1-administered mouse, gene repair effect was obtained, but little. A ratio of gene normalization in both liver lobes of a preparation 3-administered homotransgenic mouse was 8.7% in a liver lobe to which a preparation had been administered, and 0% in a liver lobe to which no preparation had been administered. Therefore, about 9% gene repair effect was obtained. In addition, as a result of mass spectrometry, a peak at 13,672Da of ATTR V30M was recognized in an untreated transgenic mouse (Fig.4A), while in addition to a peak at 13,672Da, a peak at 13,640Da (Fig. 4B, ↓) shifted to molecular weight 32Da minus due to repair of Met to Val was recognized in a preparation 3-administered transgenic mouse. These results show that, by directly administering a DNA oligonucleotide embedded in atelocollagen to a liver, an abnormal transthyretin gene was repaired, and normal transthyretin was produced, and it can be said that the DNA olignonucleotide embedded in atelocollagen of the present invention has effect of treating FAP.

Example 4

Gene conversion rate in HepG2 cell (2)

1) Preparation of gene conversion
As a DNA oligonucleotide, three kinds of 51mer YKS-384 (SEQ ID NO: 7), YKS-382 in which 1, 2, 3, 47, 49 and 50 positions in SEQ ID NO: 7 were substituted with Locked nucleic acid (trade name) (LNA), and YKS-383 in which 1, 2, 3, 10, 11, 12, 14, 34, 35, 38, 47, 49 and 50 positions in SEQ ID NO: 7 were substituted with Locked nucleic acid (trade name) (LNA) were synthesized. The oligonucleotides were designed based on a human TTR gene. According to the method described in Preparation Example 1, a solution preparation containing an associated body of the above three kinds of oligonucleotides and collagen (DNA oligonucleotide (10μM) embedded in 0.5% atelocollagen) was prepared.

2) Study of gene conversion rate in HepG2 cell
On the previous day, $2 \times 10^5$ HepG2 cells (purchased from Dainippon Pharmaceutical Co., Ltd.) were seeded on a 12-well plate, and transfected with each 600μl of a DNA oligonucleotide 51mer (10μM) embedded in 0.5% atelocollagen prepared in the 1), and cells were recovered after 6 days. As a control, a solution of a DNA oligonucleotide (YKS-384 (SEQ ID NO: 7)) alone was prepared, and transfection was performed similarly using this solution. A DNA was extracted from recovered cells, and a gene conversion rate was calculated using a MASA method (mutant allele specific amplification) and real time PCR designed so as to effectively amplify only an abnormal allele (ATTR Val30Met) using a mutated DNA-specific oligonucleotide designed so that a base corresponding to a mutated sequence becomes a 3'-terminus.

Based on the aforementioned experimental results, a gene conversion rate was confirmed, and the rate was found to be 4% in the case that YKS-384 was used as a DNA oligonucleotide in a DNA oligonucleotide embedded in atelocollagen, 10% in the case of YKS-382 containing 6 LNAs, and 23% in the case of YKS-383 containing 13 LNAs. Gene conversion did not occur in the case of YKD-384 alone.

Example 5

Study of conversion rate of bases encoding 50th and 114th amino acids conversion on TTR gene in HepG2 cell by DNA oligonucleotide

By study in Example 1, it was made clear that, by using a DNA oligonucleotide embedded in atelocollagen, conversion of bases encoding 30th amino acid on a TTR gene in HepG2 cells can be promoted as compared with use of a DNA oligonucleotide alone. Therefore, it was studied that conversion of bases encoding other amino acid on a TTR gene which is cause of FAP can be promoted by using a DNA oligonucleotide embedded in atelocollagen.

1) Preparation for facilitating gene conversion
In order to study effect of normalizing genes of FAP ATTR Ser50 Ile in which 50th serine of a transthyretin gene is mutated into isoleucine, and FAP ATTR Tyr 114 Cys in which 114th tyrosine is mutated to cysteine, a DNA oligonucleotide 74mer (SEQ ID NOs: 8 and 9, in which bases to be gene-converted are disposed at a center, and 3 bases at both ends are phosphorothioateoligonucleotide) as a DNA oligonucleotide was synthesized. According to the method described in Preparation Example 1, a solution preparation containing an associated body of the aforementioned two kinds of DNA oligonucleotides and collagen (DNA oligonucleotide (10μM) in 0.5% atelocollagen) was prepared.

2) Study of gene conversion rate in HepG2 cell
On the previous day, $2 \times 10^5$ HepG2 cells were seeded on a 12-well plate, and transfected with each 600μl (culture solution 600μl) of a DNA oligonucleotide (10μM 74mer) embedded in 0.5% atelocollagen prepared in 1) and a DNA oligonucleotide (10μM 74mer), and cells were recovered after 5 days. A DNA was extracted from recovered cells, and a gene conversion rate was calculated using a MASA method and real time PCR.
A DNA oligonucleotide (SEQ ID NO: 8) designed so as to convert bases encoding 50th amino acid on a human transthyretin gene can be converted, and a DNA oligonucleotide (SEQ ID NO: 9) designed so that bases encoding 114th amino acid on a human transthyretin gene were added to HepG2 cells as a DNA oligonucleotide embedded in atelocollagen (atelocollagen concentration: 0.5%, DNA oligonucleotide concentration :10μM) and a DNA oligonucleotide alone, respectively. As a result, in a DNA oligonucleotide designed so that bases encoding 50th amino acid on a human transthyretin gene can be converted, a base conversion rate was 0% in the case of a DNA oligonucleotide, and 1.61% in the case of a DNA oligonucleotide embedded in atelocollagen and, in a DNA oligonucleotide designed so that bases encoding 114th amino acid on a human transthyretin gene can be converted, a base conversion rate was 0% in the case of a DNA oligonucleotide alone, and 0.58% in the case of a DNA oligonucleotide embedded in atelocollagen. This result shows that the DNA oligonucleotide embedded in atelocollagen of the present invention can promote base conversion by a DNA oligonucleotide even when a base mutation site is different. Further, this shows that the present invention can provide a therapeutic to different type FAP.

Example 6

Study of gene conversion rate in human retina epithelial cell

By study in Example 1, it was made clear that, by using a DNA oligonucleotide embedded in atelocollagen, conversion of bases encoding 30th amino acid on a TTR gene in HepG2 cells can be promoted as compared with use of a DNA oligonucleotide alone. On the other hand, since in FAP, abnormal TTR is produced not only in liver, but also retina, it was studied that conversion of bases on a TTR gene can be promoted by using a DNA oligonucleotide embedded in atelocollagen, also in human retina cells.

1) Preparation for facilitating gene conversion
Regarding a DNA oligonucleotide (SEQ ID NO: 4) based on a human TTR gene, according to the method described in Preparation Example 1, a solution preparation containing an associated body of a DNA oligonucleotide and collagen (DNA oligonucleotide (10μM) embedded in 0.5%, 0.25%, 0.1% atelocollagen) was prepared.

2) Study of gene conversion rate in human retina epithelial cell
On the previous day, $2 \times 10^5$ cells of a human retina epithelial cell (ARPE19 strain) were seeded on a 12-well plate, and transfected with each 600μl (culture solution 600μl) of a DNA oligonucleotide (10 μM 74mer) embedded in 0.1%, 0.25% or 0.5% atelocollagen, and cells were recovered after 5 days. A DNA was extracted from recovered cells, and a gene conversion rate was calculated using a MASA method and real time PCR.

When a DNA oligonucleotide embedded in atelocollagen was added to a human retina epithelial cell, a base conversion rate was 0% at an atelocollagen concentration of 0.1%, 5.91% at 0.25% and 2.08% at 0.5%. This result shows that the DNA oligonucleotide embedded in atelocollagen of the present invention can promote base conversion by a DNA oligonucleotide not only in human liver cells but also in human retina cells.

Example 7

Study of conversion rate of gene mutation associated with Fabry's disease

1) Preparation of gene conversion

Based on mutation of an $\alpha$-glactosidase gene seen in Fabry's disease, a DNA oligonucleotide (SEQ ID NO: 10 or 11, in which bases to be gene-converted are disposed at a center, and 3 bases at both ends are phosphorothioateoligonucleotide) for converting 125th or 374th amino acid was synthesized as a DNA oligonucleotide. According to the method described in Example 6, a solution preparation containing an associated body of the aforementiond 3 kinds of oligonucleotides and collagen (DNA oligonucleotide (10$\mu$M) embedded in 0.1%, 0.25%, 0.5% atelocollagen) was prepared.

2) Study of gene conversion rate in Fabry's disease patient-derived human fibroblast

Fabry's disease patient-derived human fibroblast which had been seeded on the previous day so that a cell density became about 50% on the day of administration of preparation was seeded on a 12-well plate, and transfected with each 600$\mu$l (cultured solution 600$\mu$l) of a DNA oligonucleotide (10$\mu$M 74mer) embedded in 0.1%, 0.25% or 0.5% atelocollagen, and cells were recovered after 7 days. A DNA was extracted from recovered cells, and a gene conversion rate was calculated using a MASA method and real time PCR.

When a DNA oligonucleotides embedded in each of 3 kinds of atelocollagens was added to a human fibroblast, a base conversion rate was as follows:

DNA oligonucleotide (SEQ ID NO: 10) for converting 125th amino acid
Atelocollagen concentration: base conversion rate
0.1%: 0%, 0.25%: 0%, 0.5%: 0.95%
DNA oligonucleotide (SEQ ID NO: 11) for converting 374th amino acid
Atelocollagen concentration: base conversion rate
0.1%: 1.3%, 0.25%: 0%, 0.5%: 0%

This result shows that the DNA oligonucleotide embedded in atelocollagen of the present invention can promote base conversion by a DNA oligonucleotide also to gene mutation associated with Fabry's disease. Further, this shows that the present invention can provide a therapeutic for Fabry's disease.

Reference Example 1

Assessment of suitability of gene conversion rate calculated by real time PCR utilizing a MASA method

1) Using a DNA extracted from HepG2 cells to which a DNA oligonucleotide (10$\mu$M 74mer) embedded in 0.5% atelocollagen had been added in 5) of Example 1, an exon 2 (exon at mutated site) of a TTR gene was amplified, and transformed into DH5$\alpha$ cells. A plasmid DNA was purified from the resulting colony, and cut with a restriction enzyme Nsi 1, whereby, a rate of conversion from a normal TTR gene to an ATTR Val30Met gene was studied. As a result, in 50 colonies of 60 colonies, gene conversion was recognized (8.3%, and approximately the same result as that (10%) of real time PCR utilizing a MASA method was obtained.)

2) A 100%, 10% or 1% ATTR Val30Met gene obtained by diluting a DNA obtained from a FAP ATTR Val30Met homozygote patient with a DNA of a normal person was used as a standard, real time PCR utilizing a MASA method was performed regarding a 3.125% ATTR Val30Met gene, and correlation between a theoretical value and a measured value was studied. As a result, a theoretical value and a calculated value show primary order correlation, and better correlation of a correlation coefficient of 0.9956 was recognized.

3) In order to deny a possibility that, in real time PCR, a DNA oligonucleotide remaining in a system functions as a template to produce a PCR product and, as a result, calculation is performed as if gene conversion occurred, real time PCR was performed using a DNA oligonucleotide (SEQ ID NO:4) as a template. As a result, since when a DNA oligonucleotide was used as a template, a mild increasing curve is seen from an initial stage, and a Tm value of a PCR product by melting curve analysis is entirely different from that of a standard gene (ATTR Va130Met), it was made clear that a DNA oligonucleotide does not function as a template.

From the foregoing results, suitability of a gene conversion rate calculated by real time PCR utilizing a MASA method was confirmed.

INDUSTRIAL APPLICABILITY

According to the present invention, there are provided a preparation for facilitating gene conversion for site-specifically converting a specific base pair present on a genome gene of a cell, and a preparation for gene therapy. The preparation of the present invention is biodegradable and of low antigenicity and, by using collagen whose high safety has been already confirmed in the case of administration to a living body, an oligonucleotide can be introduced into a cell at an extremely high efficiency, and can be localized in a nucleus, and conversion of a genome gene can be promoted effectively. The preparation of the present invention is useful also as a preparation for facilitating an oligonucleotide localization in a nucleus. By using these preparations, gene therapy, and production of gene-mutated animal and plant are possible.

**Claims**

1. A preparation for facilitating site-specific gene conversion, comprising at least a collagen and an oligonucleotide for gene conversion.

2. A preparation for site-specific gene therapy, comprising at least a collagen and an oligonucleotide for gene conversion.

3. The preparation according to claim 1 or 2, wherein collagen is water-soluble collagen.

4. The preparation according to claim 3, wherein the water-soluble collagen is atelocollagen.

5. The preparation according to any one of claims 1 to 4, wherein the oligonucleotide for gene conversion is an oligonucleotide comprising of at least 20 bases.

6. The preparation according to any one of claims 1 to 5, wherein the oligonucleotide for gene conversion is a RNA/ DNA chimeric oligonucleotide or a DNA oligonucleotide.

7. The preparation according to claim 5 or 6, wherein the oligonucleotide for gene conversion is an oligonucleotide having a nucleotide sequence forming a Watson-Crick type base pair containing mismatch pairing of 1 to 3 base pairs, with a sense strand or an antisense strand of a gene to be converted.

8. The preparation according to claim 5 or 6, wherein the oligonucleotide for gene conversion is an oligonucleotide having a nucleotide sequence forming a Watson-Crick type base pair containing deletion or insertion of 1 to 3 bases, with a sense strand or an antisense strand of a gene to be converted.

9. The preparation according to claim 7, wherein the mismatch pairing is located at a central part of an oligonucleotide.

10. The preparation according to claim 8, wherein the deletion or insertion of bases is located at a central part of an oligonucleotide.

11. The preparation according to any one of claims 1 to 10, wherein a dosage form is solution-like.

12. The preparation according to claim 11, which contains a phosphate salt in a range of 0.01 M to 0.1M.

13. The preparation according to claim 11, which contains a sodium salt in a range of 0.07M to 0.14M.

14. The preparation according to any one of claims 11 to 13, wherein an oligonucleotide for gene conversion and a collagen form a particulate associated body.

15. The preparation according to claim 14, wherein a long diameter of the particulate associated body is 300nm to 50μm.

16. The preparation according to any one of claims 11 to 15, which comprises collagen in a range of 0.01 to 1.0% by weight.

17. The preparation according to any one of claims 11 to 15, which comprises collagen in a range of 0.01 to 0.25% by weight.

18. A preparation for facilitating site-specific gene conversion or a preparation for gene therapy, obtained by dissolving collagen in a solution containing 0.01M to 0.1M of a phosphate salt and 0.07M to 0.14M of a sodium salt, adding an oligonucleotide solution for gene conversion containing the same concentration of a phosphate salt and the same concentration of a sodium salt thereto, and stirring this under a temperature of 1 to 10°C.

19. The preparation according to any one of claims 1 to 10, wherein a dosage form is solid-like, and an oligonucleotide for gene conversion and a collagen form a particulate associated body.

20. The preparation according to claim 19, wherein an oligonucleotide for gene conversion and a collagen form a particulate associated body.

21. The preparation according to claim 20, wherein a long diameter of a particulate associated body is 300nm to 50μm.

22. A method of arbitrarily converting a specific base on a genome gene in a nucleus of a cell, which comprises contacting the preparation for facilitating gene conversion as defined in any one of claims 1, and 3 to 21 with the cell.

23. The method according to claim 22, wherein the cell is a mammal cell.

24. The method according to claim 22, wherein the cell is yeast or fungus.

25. A preparation for facilitating an oligonucleotide intranuclear localization in a nucleus, comprising at least a collagen and an oligonucleotide.

26. The preparation for facilitating intranuclear localization according to claim 25, which contains a phosphate salt in a range of 0.01 M to 0.1M.

27. The preparation for facilitating intranuclear localization according to claim 25 or 26, which contains a sodium salt in a range of 0.07M to 0.14M.

28. The preparation for facilitating intranuclear localization according to any one of claims 25 to 27, wherein the oligonucleotide and the collagen form a particulate associated body.

29. The preparation for facilitating intranuclear localization according to claim 28, wherein a long diameter of the particulate associated body is 300nm to 50 μm.

30. The preparation for facilitating intranuclear localization according to any one of claims 25 to 29, which comprises a collagen in a range of 0.01 to 1.0% by weight.

31. The preparation for facilitating intranuclear localization according to any one of claims 25 to 29, which comprising collagen in a range of 0.05 to 0.25% by weight.

32. A method of gene conversion of a cell, which comprises contacting a composition comprising at least collagen and an oligonucleotide for gene conversion with a cell in a living body by oral, nasal, via lung, intraportal, intramuscular, subcutaneous, organ surface, intraorgan or transdermal administration.

33. A method of treating a gene diseases, which comprises using the method as defined in claim 32.

# Fig 1

(a)

Normal TTR gene

(b)  5'-T*G*A* ACA CAT GCA TGG CCA CAT TG*A* T*-3'

(C)  5'-G*C*A* GCC TTT CTG AAC ACA TGC ATG GCC ACA TTG ATG GCA GGA C*T*G* -3'

(d)· 5'-T*C*C* CAG GTG TCA TCA GCA GCC TTT CTG AAC ACA TGC ATG GCC ACA TTG ATG GCA GGA CTG CCT CGG ACA GCA* T*C*-3'

Fig 2

(a) DNA oligonucleotide embedded in 0.01% atelocollagen
(b) DNA oligonucleotide embedded in 0.1% atelocollagen
(c) DNA oligonucleotide encapsulated in HVJ-liposome

Fig 3

(a) DNA oligonucleotide

(b) DNA oligonucleotide embedded in 0.05% atelocollagen

Fig. 4

A

B

<div align="center">

**INTERNATIONAL SEARCH REPORT**

</div>

| | International application No. |
|---|---|
| | PCT/JP03/11962 |

| A. CLASSIFICATION OF SUBJECT MATTER |
|---|
| Int.Cl⁷ A61K48/00, 31/7088, 47/42, 9/08, 9/14, A61P43/00 |

According to International Patent Classification (IPC) or to both national classification and IPC

| B. FIELDS SEARCHED |
|---|
| Minimum documentation searched (classification system followed by classification symbols) |
| Int.Cl⁷ A61K48/00, 31/70-31/7088, 47/00-47/42, 9/00-9/14, A61P1/00-43/00 |

Documentation searched other than minimum documentation to the extent that such documents are included in the fields searched

Electronic data base consulted during the international search (name of data base and, where practicable, search terms used)
MEDLINE(STN), CAPLUS(STN), EMBASE(STN), BIOSIS(STN), BIOTECHABS(STN), JSTPLUS(JOIS), WPI(DIALOG), DDBJ/GenBank/EMBL, SwissProt/PIR/PDB, GeneSeq

| C. DOCUMENTS CONSIDERED TO BE RELEVANT | | |
|---|---|---|
| Category* | Citation of document, with indication, where appropriate, of the relevant passages | Relevant to claim No. |
| X<br>Y | EP 1295611 A1 (Sumitomo Pharmaceuticals Co., Ltd.),<br>19 June, 2001 (19.06.01),<br>Claims; examples; Par. No. [0035]<br>& WO 01/97857 A1 & AU 200164317 A | 25-31<br>1-21 |
| X<br>Y | WO 01/34206 A2 (CMIC CO., LTD.),<br>17 May, 2001 (17.05.01),<br>Claims; examples<br>& JP 2001-199903 A & AU 200113034 A<br>& EP 1229941 A2 & BR 200015419 A<br>& KR 2002060733 A & CN 1390141 A | 25-31<br>1-21 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 17 October, 2003 (17.10.03) | 04 November, 2003 (04.11.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

### INTERNATIONAL SEARCH REPORT

| | International application No. |
|---|---|
| | PCT/JP03/11962 |

C (Continuation). DOCUMENTS CONSIDERED TO BE RELEVANT

| Category* | Citation of document, with indication, where appropriate, of the relevant passages | Relevant to claim No. |
|---|---|---|
| Y | WO 95/15972 A1 (THOMAS JEFFERSON UNIVERSITY), 15 June, 1995 (15.06.95), Claims; examples<br>& JP 9-506511 A     & AU 9513995 A<br>& EP 733059 A1     & NZ 278490 A<br>& CN 1215755 A     & CN 1142829 A | 1-21,25-31 |
| Y | WO 97/48714 A1 (THOMAS JEFFERSON UNIVERSITY), 24 December, 1997 (24.12.97), Claims; examples<br>& JP 2000-512853 A   & EP 968224 A1<br>& US 5731181 A     & US 5795972 A<br>& AU 9734920 A     & CN 1222158 A<br>& NZ 333201 A     & KR 2000011075 A | 1-21,25-31 |
| Y | Yukio ANDO et al., "RNA/DNA Chimeraoligonucleotide ni yoru Kazokusei Amyloidopolyneuropathy(FAP) no Idenshi Chiryo no Kisoteki Kenkyu", Kosei Kagaku Kenkyuhi Hojokin Tokutei Shikkan Taisaku Kenkyu Jigyo Amyloidosis ni Kansuru Kenkyu Heisei 13 Nendo Sokatsu Kenkyu Hokokusho, 2002 March, pages 28 to 30 | 1-21,25-31 |
| Y | GAMPER, H.B. et al, The DNA strand of chimeric RNA/DNA oligonucleotides can direct gene repair/conversion activity in mammalian and plant cell-free extracts., Nucleic Acids Research, 2000, 28(21), pages 4332 to 4339; full text | 1-21,25-31 |
| Y | KREN, B.T. et al., Correction of the UDP-glucuronosyltrans-ferase gene defect in the Gunn rat model of Crigler-Najjar syndrome type I with a chimeric oligonucleotide., Proc.Natl.Acad.Sci., USA, 1999, 96, pages 10349 to 10354; full text | 1-21,25-31 |
| Y | Yasushi KANEDA, "Idenshi Chiryo to Sonogo no Shinpo Idenshi Donyuho -Liposome-", Biotherapy, 1994, 8(10), pages 1265 to 1272; page 1266, right column, line 12 to page 1267, right column, last line; Fig. 3 | 25-31 |
| Y | Yasushi KANEDA, "Seitainai Soshiki eno Chokusetsuteki Idenshi Donyu, Experimental Medicine, 1994, 12(2), pages 184 to 192; page 185, lines 2 to 8; Fig. 1 | 25-31 |
| P,Y | TAUBES, G., The Strange Case of Chimeraplasty., Science, 2002, 298, pages 2116 to 2120 | 1-21,25-31 |
| A | BENSON, M.D. Familial amyloydotic polyneuropathy., Trends in Neurosciences, 1989, 12, pages 88 to 92 | 1-21,25-31 |

Form PCT/ISA/210 (continuation of second sheet) (July 1998)

**EP 1 550 463 A1**

| INTERNATIONAL SEARCH REPORT | International application No. |
|---|---|
| | PCT/JP03/11962 |

C (Continuation).   DOCUMENTS CONSIDERED TO BE RELEVANT

| Category* | Citation of document, with indication, where appropriate, of the relevant passages | Relevant to claim No. |
|---|---|---|
| A | BRAASCH, D.A. et al., Novel Antisense and Peptide Nucleic Strategies for Controlling Gene Expression., Biochemistry, 2002, 41(14), pages 4503 to 4510 | 1-21,25-31 |

Form PCT/ISA/210 (continuation of second sheet) (July 1998)

26

## INTERNATIONAL SEARCH REPORT

| International application No. |
|---|
| PCT/JP03/11962 |

**Box I   Observations where certain claims were found unsearchable (Continuation of item 2 of first sheet)**

This international search report has not been established in respect of certain claims under Article 17(2)(a) for the following reasons:

1. ☒ Claims Nos.: 22-24, 32, 33

   because they relate to subject matter not required to be searched by this Authority, namely:
   The inventions according to these claims involve a method of administering a composition having a gene to the human body and thus pertain to methods for treatment of the human body by therapy (PCT Article 17(2)(a)(i) and PCT Rule 39.1(iv)).

2. ☐ Claims Nos.:

   because they relate to parts of the international application that do not comply with the prescribed requirements to such an extent that no meaningful international search can be carried out, specifically:

3. ☐ Claims Nos.:

   because they are dependent claims and are not drafted in accordance with the second and third sentences of Rule 6.4(a).

**Box II   Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**   ☐   The additional search fees were accompanied by the applicant's protest.

☐   No protest accompanied the payment of additional search fees.

Form PCT/ISA/210 (continuation of first sheet (1)) (July 1998)

SEQUENCE LISTING

<110> Sumitomo Pharmaceuticals Co., Ltd.
Koken Co., Ltd.

<120> An agent for accelerating site directed gene conversion
and a therapeutic agent of genetic disease

<130> L1394 EP

<150> JP 2002-274926
<151> 2002-9-20

<160> 11

<170> PatentIn Ver. 2.1

<210> 1
<211> 68
<212> DNA
<213> Artificial Sequence

<220>
<222> (29)..(39)
<223> Description of Artificial Sequence:2'-O-methyl-RNA

<220>
<222> (45)..(54)
<223> Description of Artificial Sequence:2'-O-methyl-RNA

<400> 1
atcaatgtgg ccatgcatgt gttcattttu gaacacaugc atggccacau ugaugcgcgt    60
tttcgcgc                                                             68

<210> 2
<211> 25
<212> DNA
<213> Artificial Sequence

<220>
<222> (1)..(3)
<223> Description of Artificial Sequence:Phosphorothioate linkage

<220>
<222> (23)..(25)
<223> Description of Artificial Sequence:Phosphorothioate linkage

<400> 2
tgaacacatg catggccaca ttgat                                          25

<210> 3
<211> 45
<212> DNA
<213> Artificial Sequence

<220>
<222> (1)..(3)
<223> Description of Artificial Sequence:Phosphorothioate linkage

<220>
<222> (43)..(45)
<223> Description of Artificial Sequence:Phosphorothioate linkage

<400> 3
gcagcctttc tgaacacatg catggccaca ttgatggcag gactg                    45

<210> 4
<211> 74
<212> DNA
<213> Artificial Sequence

<220>
<222> (1)..(3)
<223> Description of Artificial Sequence:Phosphorothioate linkage

<220>
<222> (72)..(74)
<223> Description of Artificial Sequence:Phosphorothioate linkage

<400> 4
tcccaggtgt catcagcagc ctttctgaac acatgcatgg ccacattgat ggcaggactg    60
cctcggacag catc    74


<210> 5
<211> 74
<212> DNA
<213> Artificial Sequence

<220>
<222> (1)..(3)
<223> Description of Artificial Sequence:Phosphorothioate linkage

<220>
<222> (72)..(74)
<223> Description of Artificial Sequence:Phosphorothioate linkage

<400> 5
tcccaggatc cctcagaggt ctttttgaac actttcacag ccacgtctac agcagggctg    60
cctcggacag catc    74



<210> 6
<211> 74
<212> DNA
<213> Artificial Sequence

<220>
<222> (1)..(3)
<223> Description of Artificial Sequence:Phosphorothioate linkage

<220>
<222> (72)..(74)
<223> Description of Artificial Sequence:Phosphorothioate linkage

<400> 6
tcccaggtct catcagcagc ctttttgaac acgtgcatag acacgtcgac tgcaggactg    60
cctcggacgg catc    74


<210> 7
<211> 51
<212> DNA
<213> Artificial Sequence

<220>
<223> Description of Artificial Sequence:DNA oligonucleotide

<400> 7
tcagcagcct ttctgaacac atgcatggcc acattgatgg caggactgcc t    51

<210> 8
<211> 74
<212> DNA
<213> Artificial Sequence

<220>
<222> (1)..(3)
<223> Description of Artificial Sequence:Phosphorothioate linkage

<220>
<222> (72)..(74)
<223> Description of Artificial Sequence:Phosphorothioate linkage

<400> 8
ctcctcagtt gtgagcccat gcagctctcc agactcaatg gttttcctat aaggtgtgaa     60
agtctggatt aagt                                                        74

<210> 9
<211> 74
<212> DNA
<213> Artificial Sequence

<220>
<222> (1)..(3)
<223> Description of Artificial Sequence:Phosphorothioate linkage

<220>
<222> (72)..(74)
<223> Description of Artificial Sequence:Phosphorothioate linkage

<400> 9
cttgggattg gtgacgacag ccgtggtgga ataggagcag gggctcagca gggcggcaat     60
ggtgtagcgg cggg                                                        74

<210> 10
<211> 74
<212> DNA
<213> Artificial Sequence

<220>
<222> (1)..(3)
<223> Description of Artificial Sequence:Phosphorothioate linkage

<220>
<222> (72)..(74)
<223> Description of Artificial Sequence:Phosphorothioate linkage

<400> 10
gcagtcaagg ttgcacatga agcgctccca gtgcagccag cccatggtag gcgtccttgc     60
caatccattg tcca                                                        74

<210> 11
<211> 74
<212> DNA
<213> Artificial Sequence

<220>
<222> (1)..(3)
<223> Description of Artificial Sequence:Phosphorothioate linkage

<220>
<222> (72)..(74)
<223> Description of Artificial Sequence:Phosphorothioate linkage

<400> 11

```
gccatgatag cccagagggc catctgagtt acttgctgat tccagctgag gccaaagttg    60
ccaatcacta actg                                                       74
```